Europäisches Patentamt

European Patent Office

Office européen des brevets

(19)

(11) Veröffentlichungsnummer : **0 080 203**
**B1**

# (12) EUROPÄISCHE PATENTSCHRIFT

(45) Veröffentlichungstag der Patentschrift :
26.06.85

(21) Anmeldenummer : **82110781.0**

(22) Anmeldetag : **22.11.82**

(51) Int. Cl.⁴ : **A 61 L   2/26**

(54) **Sterilisierbehälter.**

(30) Priorität : **23.11.81 DE 3146349**

(43) Veröffentlichungstag der Anmeldung :
**01.06.83 Patentblatt 83/22**

(45) Bekanntmachung des Hinweises auf die Patenterteilung : **26.06.85 Patentblatt 85/26**

(84) Benannte Vertragsstaaten :
**AT CH FR GB IT LI**

(56) Entgegenhaltungen :
**CH-A-   164 675
DE-A- 2 207 339
DE-A- 2 301 144
DE-A- 2 603 912
DE-A- 2 610 290
DE-B- 1 116 869
DE-B- 1 217 550
FR-A- 2 375 869**

(73) Patentinhaber : **Georg Wagner KG
Schulstrasse 16a
D-8000 München 19 (DE)**

(72) Erfinder : **Lorenz, Jürgen W.
Helterwangerstrasse 20
D-8000 München 70 (DE)**

(74) Vertreter : **Koch, Günther, Dipl.-Ing. et al
Kaufingerstrasse 8
D-8000 München 2 (DE)**

**Beschreibung**

Die Erfindung bezieht sich auf einen Sterilisierbehälter der im Oberbegriff des Patentanspruchs 1 angegebenen Gattung. Derartige Sterilisierbehälter dienen der Aufnahme ärztlicher Instrumente, Textilien u. s. w., um diese einer Dampfsterilisation zu unterwerfen. Nach der Sterilisation verbleiben diese nunmehr sterilen Instrumente in diesen Behältern, in denen sie auch für die Operation bereitgestellt werden. Beim Sterilisieren wird zunächst die Luft aus dem Behälter abgesaugt, und es erfolgt dann eine mehrmalige Dampfsterilisation, wobei abwechselnd Dampf eingeblasen und abgesaugt wird. Dieser Medienaustausch erfolgt durch die Filter bzw. Ventile hindurch, wobei nach Abschluß des Sterilisiervorganges die Filter bzw. Ventile das Sterilgut gegen das Eindringen von Keimen schützen.

Sterilisierbehälter mit Filtern sind aus der DE-A-2 301 144 und der DE-A-2 207 339 bekannt. Hierbei liegt das Filter unmittelbar unter den Perforationslöchern des Behälterdeckels bzw. über dem nach innen gezogenen Boden des Unterteils des Sterilisierbehälters, wodurch die Gefahr besteht, daß das Filter im Bereich der Perforationslöcher beschädigt bzw. unwirksam gemacht wird, wenn spitze oder stumpfe Gegenstände von außen her durch die Löcher eindringen und das Filter durchstoßen. Ferner sind die Filter einer hohen Staubbelastung und einer hohen Feuchtigkeit ausgesetzt, und es können durch die Perforationen punktuelle Verunreinigungen durch Speicheltropfen, die z. B. beim Husten austreten, erfolgen.

Bei perforiertem Boden ist eine Naßentsorgung, d. h. ein Rücktransport der chirurgischen Instrumente in Desinfektionslösung im Sterilisierbehälter nicht möglich. Diese Naßentsorgung wird jedoch häufig gefordert und sie ist nur dann möglich, wenn der Behälterunterteil wasserdicht ist.

Die Gefahr der Beschädigung des Deckelfilters ist bei einem gattungsgemäßen Sterilisierbehälter beseitigt, wie dieser beispielsweise aus der DE-A-933 054 bekannt ist. Hierbei wird die Beschädigung des Deckelfilters dadurch vermieden, daß eine kappenförmige Schutzabdeckung, die hier nur in einem mittleren, stutzenartigen Abschnitt vorgesehene Perforation überdeckt, durch die der Medienaustausch stattfindet. Diese Schutzabdeckung steht nach oben über den Behälterdeckel vor und macht ein Stapeln der Behälter übereinander unmöglich. Beim Abwischen des seitlich der Kappe freiliegenden Deckelteils besteht die Gefahr, daß Keime unter die kappenförmige Schutzabdeckung gelangen, so daß für eine äußere Keimfreiheit nicht mehr garantiert werden kann. Der als Lochblech ausgeführte Zwischendeckel, der von dem dichtend auf das Unterteil aufgesetzten Behälterdeckel umschlossen ist, soll bei dem bekannten Sterilisierbehälter einen gleichmäßigen Dampfeintritt in das Innere gewährleisten, was jedoch aus physikalischen Gründen nicht möglich ist, denn die Geschwindigkeitsverteilung bleibt durch den mittleren Einlaßstutzen bedingt ungleichförmig, und die Geschwindigkeit ist im Mittelabschnitt unter dem Stutzen am größten und fällt nach den Seiten hin ab. Der bekannte Behälter weist außerdem einen perforierten Boden mit einem darübergespannten Filtertuch auf, wodurch eine Naßentsorgung unmöglich wird.

Der dem beschriebenen bekannten Sterilisierbehälter anhaftende Nachteil der sehr ungleichmäßigen Geschwindigkeitsverteilung ist bei anderen gattungsgemäßen Sterilisierbehältern vermieden, wie diese beispielsweise aus der DE-GM-A1 615 358 und 1 234 901 bekannt sind. Hier ist die dem Medienaustausch dienende Perforation über einen größeren Teil der Deckelfläche verteilt, wodurch sich eine gleichmäßigere Geschwindigkeitsverteilung ergibt. Entsprechend ist auch die kappenförmige Schutzabdeckung weiter nach außen gezogen, sie endet jedoch vor dem Rand des Behälterdeckels und beläßt einen ringsum laufenden, freien Deckelrand, so daß sich die gleichen Nachteile hinsichtlich einer Filterverunreinigung beim Abwischen oder durch seitlichen Luftzug ergeben. Außerdem besteht neben der Gefahr der Kondensatbildung im Behälterinneren auch hier die Gefahr einer zusätzlichen Kondensateinregung durch das Deckelfilter. Diese zusätzlichen Kondensatmengen erschweren die Vakuumnachtrocknung des Sterilgutes im Sterilisator erheblich. Außerdem sind zwei Spalten vorhanden, nämlich einmal ein Spalt zwischen Behälterunterteil und Behälterdeckel, der adgedichtet werden muß, und ein weiterer Spalt zwischen Schutzabdeckung und perforiertem Deckel.

Der Zwischendeckel ist bei der DE-GM-A-1 615 358 fest am Behälterdeckel angeordnet, um das Filter unter den Perforationslöchern festzulegen. Bei dem Sterilisierbehälter nach der DE-GM-A-1 234 901 ist der Behälterdeckel zum Zwecke des Medienaustausches mit einer bis zum Rande reichenden, durchgehenden Öffnung versehen, so daß der Deckel nur aus einem Deckelring besteht, der einen Spannring aufweist, um ein die Öffnung abschließendes Filtertuch am Rand zu haltern. Diese Bauart ist in Verbindung mit modernen Sterilisieranordnungen nicht benutzbar, weil bei den hohen wechselnden Drücken das Filtertuch flächenmäßig abgestützt sein muß.

Der Erfindung liegt daher die Aufgabe zugrunde, einen gattungsgemäßen Sterilisierbehälter derart zu verbessern, daß bei allseits geschlossenem Aufbau und geschützten Ventilen bzw. Filtern der Behälternutzraum gegen die Außenluft hygienisch einwandfrei abgedichtet ist und eine sterile Lagerung des Behälterinhalts mit einer günstigen Dampfverteilung während der Sterilisation gewährleistet ist, wobei außerdem der Behälter bequem stapelbar ist.

Gelöst wird die gestellte Aufgabe durch die im Kennzeichnungsteil des Patentanspruchs 1 angegebenen Merkmale.

Dadurch, daß der Behälterdeckel den die Ventile bzw. Filter tragenden Zwischendeckel auch seitlich umschließt, kommen die Dampfdurchtrittsöffnungen an Stellen zu liegen, die weit entfernt zu dem Durchtrittsspalt liegen, der den Behälter mit der Außenluft verbindet und einen Teil des labyrinthförmigen Strömungspfades im Bereich des Deckelrandes bildet. Hierdurch sind mechanische Verletzungen des Filters bzw. der Ventile ausgeschlossen, und es wird außerdem ein wirksamer Schutz gegen das Eindringen von Nässe, Staub und Bakterien gewährleistet. Dadurch, daß der Strömungspfad seinen Eintritt bzw. Austritt seitlich unter dem Deckelflansch des Behälterdeckels hat, kann der Behälterdeckel eben gestaltet werden, so daß eine einwandfreie Stapelung möglich ist.

Durch die Merkmale des Anspruchs 2 wird erreicht, daß bei Öffnung des Behälters zugleich mit dem Behälterdeckel auch der Zwischendeckel abgenommen werden kann, wodurch der Innenraum frei zugänglich wird.

Durch die Dichtungsanordnungen gemäß den Ansprüchen 3 bis 9 wird auf einfache Weise gewährleistet, daß der Behälterinnenraum auch nach der Sterilisierung zuverlässig und bakteriendicht abgedeckt bleibt.

Nachstehend werden Ausführungsbeispiele der Erfindung anhand der Zeichnung beschrieben. In der Zeichnung zeigen :

Figur 1 einen Schnitt durch einen erfindungsgemäß ausgebildeten, mit Filter versehenen Sterilisierbehälter ;

Figur 2 in größerem Maßstab ein in einem Teilschnitt den in Fig. 1 in dem Kreis X liegenden Teil des Behälters ;

Figur 3 eine der Fig. 2 entsprechende Schnittansicht einer anderen Ausführungsform ;

Figur 4 eine weitere abgewandelte Ausführungsform in einer der Fig. 2 entsprechenden Schnittansicht ;

Figur 5 eine Schnittansicht eines Sterilisierbehälters mit einem Doppelventil zum Medienausgleich ;

Figur 6 eine auseinandergezogene perspektivische Ansicht einer weiteren Ausführungsform eines erfindungsgemäß ausgebildeten Sterilisierbehälters ;

Figur 7 eine der Fig. 2 und 3 entsprechende Teilschnittansicht des Behälters gemäß Fig. 6 ;

Figur 8 eine Teilansicht der Innenseite des Deckels gemäß Fig. 6 ;

Figur 9 eine Stirnansicht einer weiteren Ausführungsform des Behälters ;

Figur 10 einen Querschnitt des Behälters gemäß Fig. 9 ohne Zwischendeckel ;

Figur 11 in größerem Maßstab die Einzelheit Y gemäß Fig. 10 ;

Figur 12 einen Schnitt nach der Linie XII-XII gemäß Fig. 11 ;

Figur 13 eine perspektivische Teilschnittansicht des Behälters gemäß Fig. 9 bis 12 mit eingesetztem Zwischendeckel und Filtertuch ;

Figur 14 eine Teilschnittansicht des Behälters gemäß Fig. 13 ;

Figur 15 eine perspektivische Ansicht des mit Ventilen versehenen Zwischendeckels ;

Figur 16 einen Schnitt nach der Linie XVI-XVI gemäß Fig. 15, und

Figur 17 eine perspektivische Teilschnittansicht eines der Ventile des Zwischendeckels gemäß Fig. 15.

Bei allen Ausführungsbeispielen besteht der Behälter aus einem Behälterunterteil 10, einem Deckel 12 und einem von letzterem getragenen Zwischendeckel 14.

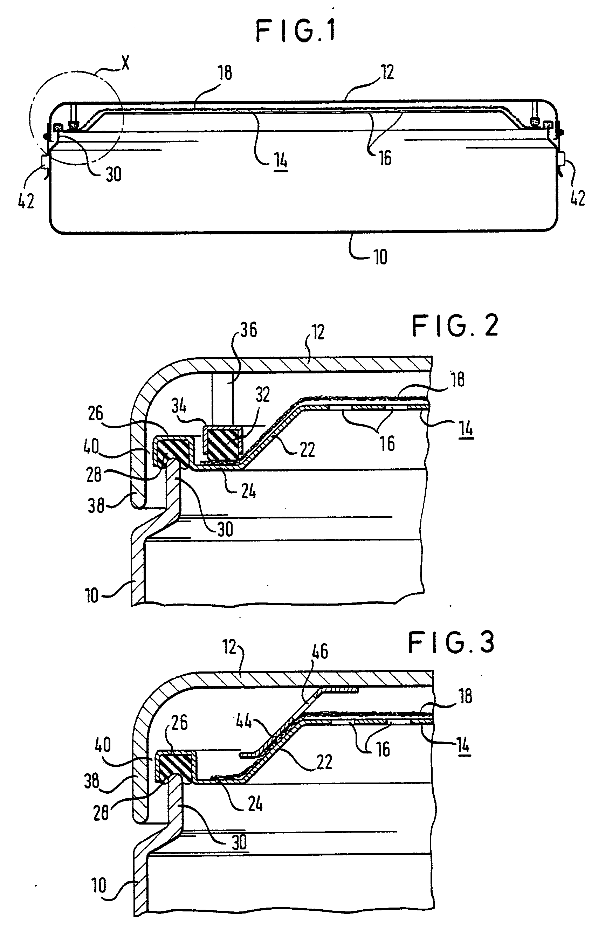

Gemäß den Ausführungsbeispielen nach Figur 1 bis Figur 4 ist der Zwischendeckel 14 perforiert und die Perforationslöcher 16 sind durch einen Filter 18 abgedeckt.

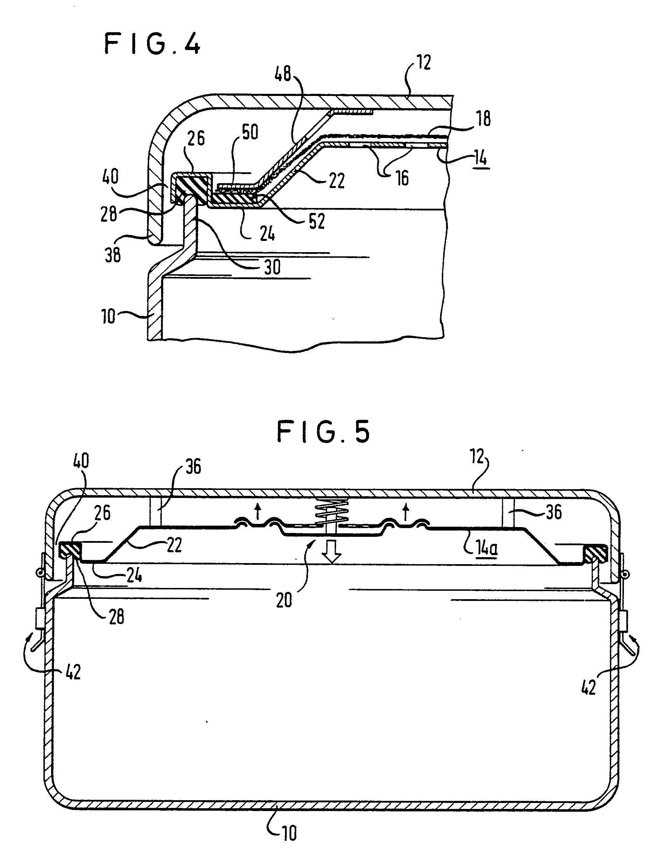

Bei dem Ausführungsbeispiel nach Figur 5 ist in dem nicht perforierten Zwischendeckel 14a ein Doppelventil 20 eingebaut, welches von der Bauart gemäß der DE-AS 12 17 550 sein kann. Stattdessen können auch zwei Ventile entsprechend der DE-AS 12 17 551 in den Zwischendeckel 14a eingebaut sein.

Der Zwischendeckel 14 geht von seinem Mittelabschnitt, der gemäß dem Ausführungsbeispiel nach Figur 1 bis 4 perforiert ist, in einem konischen Übergangsabschnitt 22 in einen Randabschnitt 24 über, der innerhalb eines U-förmigen Endabschnittes 26 einen Dichtungsring 28 trägt. Dieser Dichtungsring ruht im zusammengebauten Zustand auf dem nach innen gezogenen Rand 30 des Behälterunterteils 10. Der dampfbeständige Dichtring 28 dichtet den Behälterinnenraum hermetisch gegen die Außenluft ab. Dieser Dichtring 28 könnte auch auf dem Rand 30 des Unterteils 10 aufgesetzt sein und gegen eine entsprechende Dichtfläche des Zwischendeckels 14 wirken. Ein weiterer Dichtungsring 32 ist in ein U-Profil 34 eingesetzt, welches von Distanzstücken 36 des Deckels 12 getragen wird. Diese Distanzstücke 36 (Figur 2) sind im Abstand zueinander im Deckelinneren befestigt, so daß dazwischen der Medienaustausch gewährleistet ist. Der Zwischendeckel 14 ist mittels Schraubverbindung oder Schnellverschlüssen (in der Zeichnung nicht dargestellt) am Deckel 12 lösbar befestigt. Im verspannten Zustand drückt die Dichtung 32 den Filter 18 dichtend gegen den äußeren Randabschnitt 24. Wie insbesondere aus den Figuren 2 bis 4 ersichtlich ist, umschließt der Deckel 12 mit seinem seitlich heruntergezogenen Deckelflansch 38 seitlich den Rand 30 des Unterteils unter Belassung eines Ringspaltes 40, durch den hindurch der Medienaustausch erfolgen kann, durch den jedoch keine Gegenstände eingeführt werden können, die Filter oder Ventile beschädigen könnten. Dadurch, daß der Rand 30 des Unterteils nach innen eingezogen ist, ergibt sich ein labyrinthartiger Verschluß, der das strömende Medium durchläßt, aber das Einführen starrer Teile verhindert. Wenn der Deckel 12 mittels des Spannverschlusses 42 auf dem Unterteil festgelegt ist, dann wird der Nutzraum des Sterilisierbehälters durch den perforierten Zwischendeckel 14 in Verbindung mit der Dichtung 28 und dem Filter 18 bakteriendicht abgedeckt. Dabei wird außerdem der Dichtring 36 auf den Zwischendeckel 14 in seinem Randabschnitt 24 gedrückt. Hierdurch wird es möglich, Filter unterschiedlicher Ausführung aus Papier, Tuch und so weiter mit unterschiedlichen Dicken zu benutzen, weil durch die Elastizität des Dichtrings 32 gewisse Dickenunterschiede ausgeglichen werden können.

Bei dem Ausführungsbeispiel nach Figur 3 ist anstelle der Abstützvorrichtung 32, 34, 36 ein umlaufendes Federblech 44 am Deckel 12 festgelegt, welches in seiner Form den konischen Übergangsabschnitt 22 des Zwischendeckels entspricht. Dieses Federblech ist mit Durchtrittsöffnungen 46 für den Medienaustausch versehen und liegt bei aufgesetztem und verspannten Deckel 12 dem Filter 18 an und drückt diesen gegen den konischen Übergangsabschnitt 22.

Bei dem Ausführungsbeispiel nach Figur 4 ist am Deckel ein dem Federblech 44 ähnlicher, aber im wesentlichen starrer Rahmen 48 vorgesehen, der einen parallel zur Deckeloberfläche verlaufenden Stützrand 50 besitzt. Auf dem Randabschnitt 24 des Zwischendeckels 14 ist eine Flachdichtung 52 aufgelegt odert verklebt, und im zusammengesetzten Zustand drückt der Stützrand 50 den Rand des Filters 18 gegen die Flachdichtung 52, wodurch wiederum eine hermetische Abdichtung des Innenraumes gewährleistet ist.

Bei allen Ausführungsbeispielen erfolgt der Medienaustausch Luft-Dampf während der Sterilisation über den zwischen Deckelflansch 38 und Oberrand 30 des Behälterunterteils 10 verbleibenden Labyrinthspalt, dessen Querschnitt entsprechend dimensioniert und fixiert ist. Die über den Deckelspalt im Überdruck- bzw. Unterdruckzustand zugeführten bzw. abgeführten Medien durchströmen zwangsläufig den Filter 18 gemäß den Ausführungsbeispielen nach Figur 1 bis 4 bzw. das Ventil 20 bei dem Ausführungsbeispiel nach Figur 5. Diese Filter bzw. Ventile gewährleisten in bekannter Weise den Schutz des sterilen Behälterinhaltes gegen eine Re-Infektion. Sie sind jedoch ihrerseits durch den übergreifenden Deckel 12 vor störenden äußeren Einwirkungen geschützt.

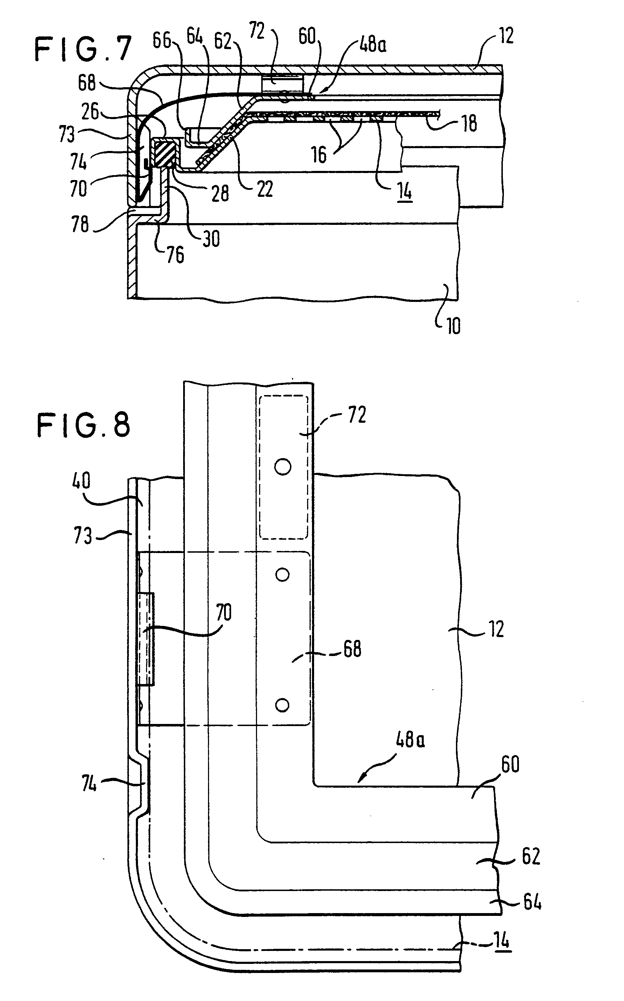

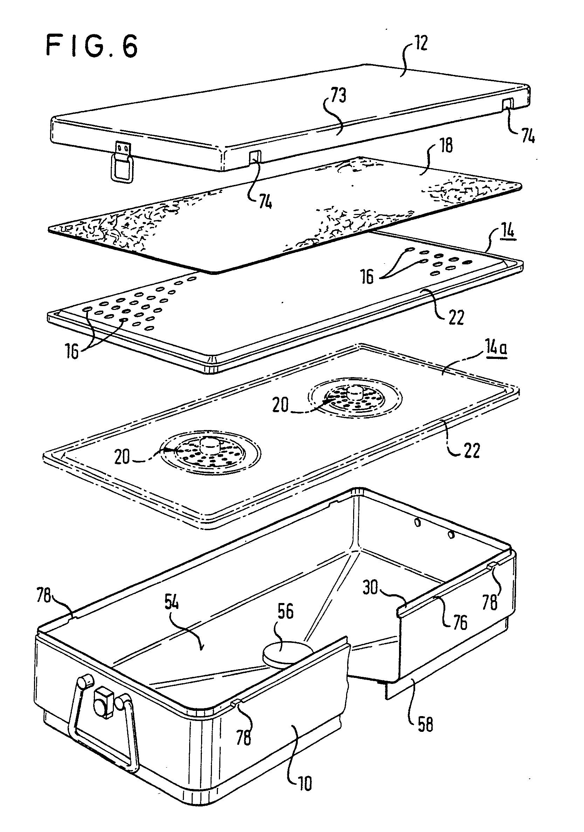

Figur 6 zeigt eine perspektivische auseinandergezogene Darstellung einer weiteren Ausgestaltung eines erfindungsgemäß ausgebildeten Sterilisierbehälters, der in seinen Einzelheiten in den Figuren 7 und 8 dargestellt ist. Abweichend von den Ausführungsbeispielen nach Figur 1 bis 5 weist der Boden 54 des Unterteils 10 in der Mitte ein Ablaßventil 56 auf, nach dem die Bodenflächen trichterförmig geneigt derart verlaufen, daß das Kondensat nach dem Ventil und durch dieses hindurch abfließen kann. Um auch bei dieser Ausführungsform einen sicheren Stand zu gewährleisten, ist das Unterteil 10 mit einem ringsum laufenden Standrand 58 versehen, wodurch einerseits der Behälter versteift und andererseits das Eindringen von Bakterien durch das Ventil verhindert wird, weil dieser Standrand das Ventil im Abstand vom Boden hält. Der Zwischendeckel 14 entspricht dem Zwischendeckel gemäß Figur 2 und findet wiederum in Verbindung mit einem Filtertuch 18 Anwendung. Der mit Ventilen 20 versehene Zwischendeckel 14a ist gegen einen Zwischendeckel 14 mit Filtertuch austauschbar. Wahlweise kann demgemäß entweder der Zwischendeckel 14 mit Filtertuch oder der Zwischendeckel 14a mit seinen Ventilen im Deckel lösbar festgelegt werden.

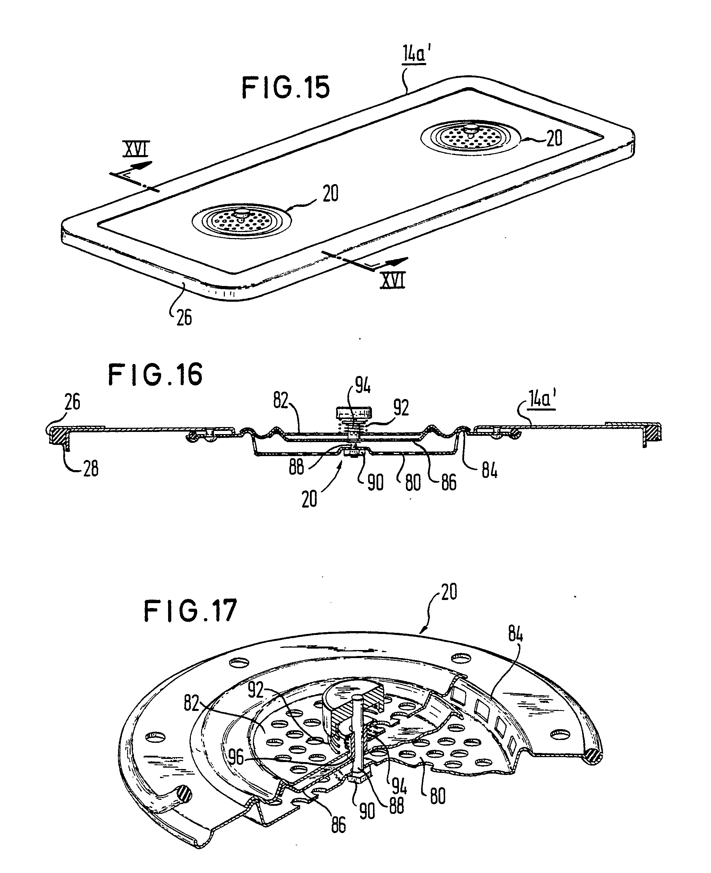

Das im Boden eingelassene Ablaßventil 56 ist zweckmäßigerweise so ausgebildet, daß es unterhalb einer Temperatur von etwa 80 bis 90 °C geschlossen, bei Sterilisationstemperatur jedoch geöffnet ist. Es weist eine Ventilscheibe auf, die von einem durch eine Schließfeder belasteten Bimetallstreifen getragen ist und in Schließstellung am Rande einer Abschlußöffnung aufliegt und gegen die Wirkung der Schließfeder beim temperaturabhängigen Umschalten des Bimetalls von diesem in die Öffnungsstellung gehoben wird. Der mit seinen abgekröpften Enden auf dem Behälterboden abgestützte Bimetallstreifen ist beiderseits der Ventilscheibe durch Führungsleisten gegen seitliche Verschiebung mit Spiel abgestützt (in der Zeichnung nicht im einzelnen dargestellt). Die Ventile 20 des Zwischendeckels 14a werden weiter unten in Verbindung mit Figur 15 und 16 beschrieben.

Wie aus Figur 7 ersichtlich, besteht der innerhalb des Deckels 12 umlaufende Rahmen 48a aus einem Blechprofil, welches einen inneren horizontalen Abschnitt 60, einen unter etwa 45° verlaufenden konischen Übergangsabschnitt 62 und einen wiederum horizontal verlaufenden äußeren am weitesten vorstehenden Abschnitt 64 aufweist, dessen äußerer Rand 66 senkrecht aufgebogen ist. Der Rahmen 48a wird innerhalb des Deckels von Blattfedern 68 getragen, die an den Seitenwänden des Deckels einerseits und andererseits an dem inneren Abschnitt 60 des Trägerrahmens 48a angeschweißt oder angenietet sind. Der an der Seitenwand des Deckels 12 anliegende Teil der Blattfeder 68 ist nach innen gebogen und bildet einen Verriegelungshaken 70, der bei eingesetztem Zwischendeckel unter dem äußeren Schenkel des U-Endabschnitts 26 des Zwischendeckels zu liegen kommt, wobei der konische Übergangsabschnitt 22 des Zwischendeckels 14 dem Übergangsabschnitt 62 des Trägerrahmens 48a anliegt. Die Verspannung des U-Endabschnitts 26 des Zwischendeckels gegen die Haken 70 wird durch Blattfedern 72 bewirkt, die mit ihrem Mittelabschnitt am Abschnitt 60 des Trägerrahmens 48 angeschweißt oder angenietet sind und sich mit ihren beiden am Ende abgerundeten Füßen am Deckel abstützen und so den Rahmen 48 vom Deckel 12 weg gemäß Figur 7 nach unten vorspannen. Die Seitenwände 73 des Deckels sind mit nach innen eingeprägten Sicken 74 versehen, die den Zwischendeckel 14 bzw. 14a im Deckel zentrieren und außerdem die Seitenwände 73 des Deckels gegenüber dem Unterteil dadurch zentrieren, daß diese Sicken 74 dem eingezogenen Rand 30 des Unterteils anliegen. Um die Seitenwände des Deckels 12 im Abstand zu dem Unterteil zu halten und den Strömungspfad 40 freizuhalten, weist die Abkröpfung 76 des oberen Randes des Unterteils im Abstand zueinander liegende Erhöhungen 78 auf, auf denen der untere Rand der Seitenwände aufliegt.

Gemäß Figur 7 ist mit dem Deckel 12 ein Zwischendeckel 14 mit Filtertuch 18 über die Haltefedern mit ihren Verriegelungshaken 70 festgeklemmt, wobei die Konusflächen 22 und 62 von Zwischendeckel 14 und Trägerrahmen 48 passend aufeinander liegen. Stattdessen könnte auch der Deckel 14a mit seinen Ventilen 20 im Deckel 12 mittels der Haltefedern 70 festgelegt werden.

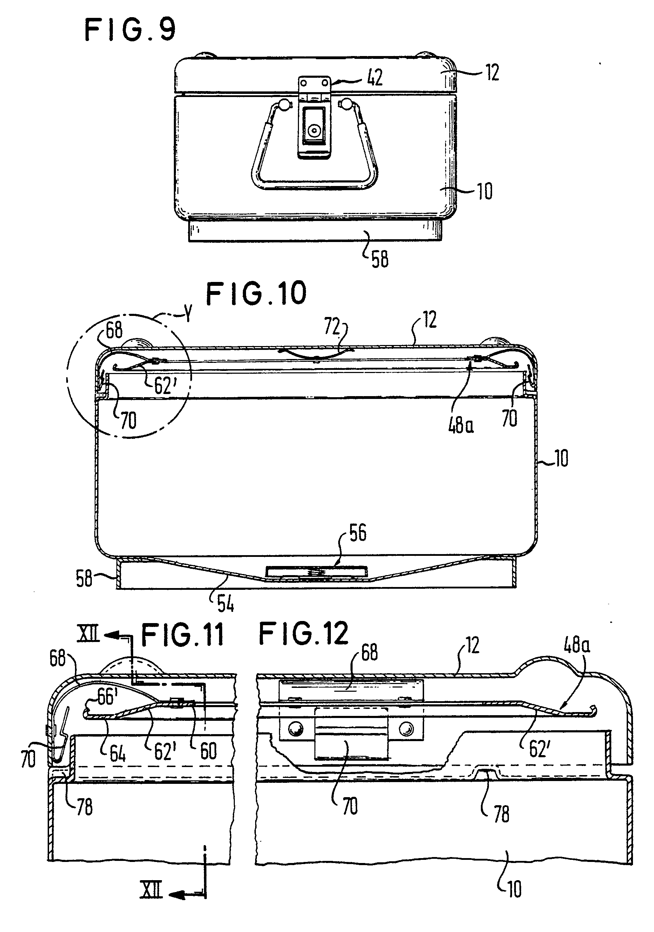

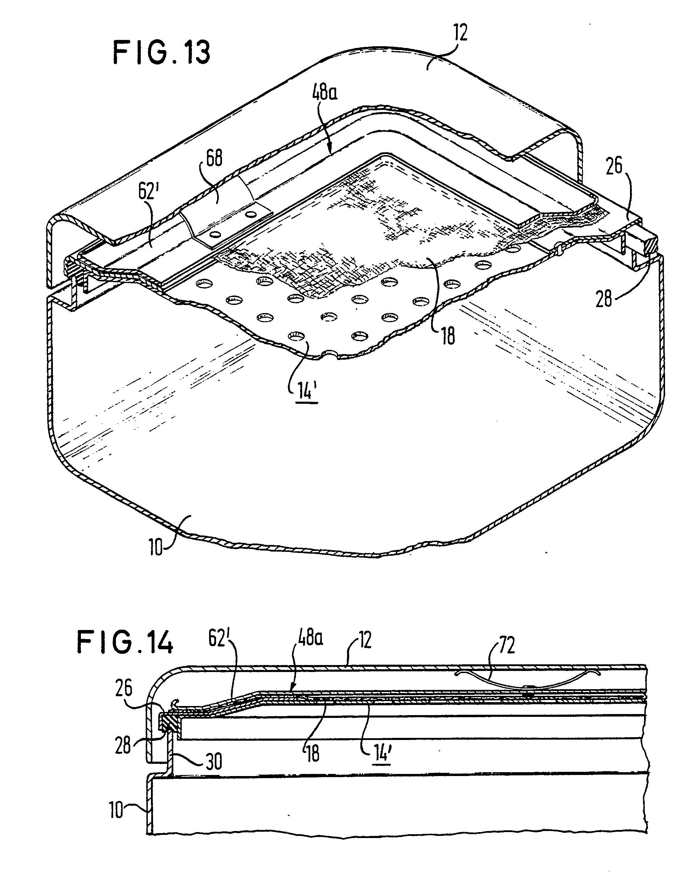

Das Ausführungsbeispiel nach Figur 9 bis 16 unterscheidet sich von den vorherigen Ausführungsbeispielen im wesentlichen dadurch, daß der Übergangsabschnitt 62' des Trägerrahmens 48a flacher ausgebildet ist in Anpassung an den Zwischendeckel 14a' gemäß Figur 15 und 16, der mit Ventilen 20 versehen und ebenso wie der Zwischendeckel 14' gemäß Figur 13 und 14 lösbar am Deckel 12 festlegbar ist, indem er gegen den Rahmen 48a gedrückt wird, so daß die Haftefeder 70 einschnappen kann.

Das Ventil 20 des Zwischendeckels 14a bzw. 14a' ist im einzelnen aus Figur 16 und 17 ersichtlich. Es ist ein als Druck- und Saugventil ausgebildetes doppelt wirkendes Verschlußventil mit einer gelochten Innenwand 80, einem gelochten äußeren Teller 82, der auf einer Wulst 84 aufruht und einem inneren Ventilteller 86. Die Teile werden durch einen Schraubbolzen 88 mit Mutter 90 zusammengehalten, auf den eine Druckschraubenfeder 92 zwischen Teller 82 und Schraubbolzenkopf aufgezogen ist. Durch diese wird der Teller 82 auf seinen Wulstsitz 84 gedrückt. Eine weitere Druckschraubenfeder 94 ist auf einen am Ventilteller 86 fixierten Zapfen 96 aufgezogen und diese Feder stützt sich zwischen dem Teller 82 und dem Kopf des Zapfens 96 ab und drückt den Ventilteller 86 nach oben gegen den Teller 82.

Durch dieses Ventil wird gewährleistet, daß der filterlose Sterilisierbehälter im Sterilisierzustand vollkommen dicht verschlossen bleibt.

**Patentansprüche**

1. Sterilisierbehälter zur Aufnahme klinischen Sterilgutes mit einem Unterteil und einem auf diesen Unterteil aufsetzbaren, mit einem herabgezogenen Flanschrand versehenen übergreifenden Behälterdeckel und mit Öffnungen zur Ermöglichung des Medienaustausches Luft-Dampf während des Sterilisiervorganges und mit die Öffnungen abdeckenden Filtern oder Ventilen, die das sterilisierte Gut während des Transportes und während der Lagerung vor einer Reinfektion schützen, wobei über den Austauschöffnungen bzw. den Ventilen oder Filtern im Abstand hierzu eine Schutzabdeckung vorgesehen und ein perforierter Zwischendeckel zwischen Behälterdeckel und Unterteil angeordnet ist, dadurch gekennzeichnet, daß die Öffnungen (16) und die Filter (18) bzw. die Ventile (20) in bzw. an dem Zwischendeckel (14 ; 14a) angeordnet sind, der auf den Behälterunterteil (10) dichtend aufsetzbar ist, daß die Schutzabdeckung von dem keinerlei Perforationen aufweisenden Behälterdeckel (12) selbst gebildet ist, und daß im Bereich des seitlich heruntergezogenen Deckelflansches (38) dieser einen solchen Abstand von dem Oberrand (30) des Unterteils (10) bzw. dem Zwischendeckel (14 ; 14a) aufweist, daß ein Ringspalt (40) gebildet ist, über den ein freier Strömungspfad in den Raum zwischen Behälterdeckel (12) und Zwischendeckel (14 bzw. 14a) führt.

2. Sterilisierbehälter nach Anspruch 1, dadurch gekenzeichnet, daß der Zwischendeckel (14 ; 14a) lösbar am Behälterdeckel (12) im Abstand zu diesem festgelegt ist.

3. Sterilisierbehälter nach den Ansprüchen 1 und 2, dadurch gekennzeichnet, daß der Zwischendeckel (14 ; 14a) einen mit Perforationen (16) bzw. Ventilen (20) versehenen Mittelabschnitt, einen konisch nach außen und unten verlaufenden übergangsabschnitt (22) und einen Randabschnitt (24) aufweist, der gegenüber dem Rand (30) des Behälterunteils (10) abgedichtet ist.

4. Sterilisierbehälter nach Anspruch 3, dadurch gekennzeichnet, daß zwischen dem Randabschnitt (24) des Zwischendeckels (14 ; 14a) und dem nach innen gezogenen Rand (30) des Behälterunterteils (10) ein Dichtring (28) angeordnet ist, der am Zwischendeckel oder am Behälterrand festgelegt ist.

5. Sterilisierbehälter nach einem der Ansprüche 1 bis 4, dadurch gekennzeichnet, daß auf dem Randabschnitt (24) des Zwischendeckels (15) ein filter (18) aufspannbar ist.

6. Sterilisierbehälter nach Anspruch 5, dadurch gekennzeichnet, daß der Behälterdeckel (12) über nach innen einstehende Distanzstücke (36) ein U-Profil (34) trägt, in dem ein Dichtring (32) verankert ist, der im zusammengebauten Zustand den Rand des Filters (18) gegen den äußeren Randabschnitt (24) des Zwischendeckels (14) drückt.

7. Sterilisierbehälter nach Anspruch 5, dadurch gekennzeichnet, daß der Behälterdeckel (12) an seiner Innenseite ein umlaufendes Federblech (44) mit Durchtrittsöffnungen (46) trägt, welches federnd gegen den konischen Übergangsabschnitt (22) des Zwischendeckels (14) und den darübergefügten Rand des Filters (18) vorgespannt ist.

8. Sterilisierbehälter nach Anspruch 5, dadurch gekennzeichnet, daß der Behälterdeckel (12) innen einen starren umlaufenden Rahmen (48) trägt, der mit einem Stützrand (50) gegen den Randabschnitt (24) des Zwischendeckels (14) unter Zwischenfügung von Filterrand und einer elastischen Flachdichtung (52) abgestützt ist.

9. Sterilisierbehälter nach Anspruch 5, dadurch gekennzeichnet, daß der unperforierte Mittelabschnitt des Zwischendeckels (14a) ein Doppelventil (20) trägt.

## Claims

1. Sterilizing container for receiving clinical sterile material comprising a lower portion and a container cover engaging thereover, adapted to be placed on said lower portion and provided with a depending flange edge, and openings for permitting the medium exchange air-vapor during the sterilizing operation and filters or valves which cover the openings and which protect the sterilizied material during transport and during storage from reinfection, a protective cover being provided over the exchange openings or valves of filters spaced therefrom and a perforated intermediate cover being disposed between the container cover and lower portion, characterized in that the openings (16) and the filters (18) or the valves (20) are disposed in or at the intermediate cover (14 ; 14a) which is adapted to be fitted sealingly on to the container lower portion (10), that the protective cover is formed by the container cover (12) itself, which does not have any perforations whatever, that in the region of the laterally depending cover flange (38) the latter is spaced from the upper edge (30) of the lower portion (10) or intermediate cover (14 ; 14a) a distance sucht that an annular gap (40) is formed via which a free flow path leads into the space between the container cover (12) and intermediate cover (14 ; 14a).

2. Sterilizing container according to claim 1, characterized in that the interdemiate cover (14 ; 14a) is detachably secured to the container cover (12) in spaced relationship therewith.

3. Sterilizing container according to claims 1 and 2, characterized in that the intermediate cover (14 ; 14a) comprises a center portion provided with perforations (16) or valves (20), a conically outwardly and downwardly extending transition portion (22) and an edge portion (24) which is sealed with respect to the edge (30) of the container lower portion (10)'

4. Sterilizing container according to claim 3, characterized in that between the edge portion (24) of the intermediate cover (14 ; 14a) and the inwardly extending edge (30) of the container lower portion (10) a sealing ring (28) is disposed which is fixed to the intermediate cover or to the container edge.

5. Sterilizing container according to any one of claims 1 to 4, characterized in that on the edge portion (24) of the intermediate cover (15) a filter (18) can be secured in tension.

6. Sterilizing container according to claim 5, characterized in that the container cover (12) carries via inwardly projecting spacers (36) a U-section (34) in which a sealing ring (32) is anchored in the assembled state presses the edge of the filter (18) against the outer edge portion (24) of the intermediate cover (14).

7. Sterilizing container according to claim 5, characterized in that the container cover (12) carries at its inner side an encircling spring sheet metal member (44) with passage openings (46) which is biased resiliently against the conical transition portion (22) of the intermediate cover (14) and the edge of the filter (18) placed thereover.

8. Sterilizing container according to claim 5, characterized in that the container cover (12) carries at the inside a rigid encircling frame (48) which bears with a support edge (50) against the edge portion (24) of the intermediate cover (14) with interposition of filter edge and a resilient flat seal (52).

9. Stezrilizing container according to claim 5, characterized in that the unperforated center portion of the intermediate cover (14a) carries a double valve (20).

## Revendications

1. Récipient de stérilisation pour la réception de matériels stériles cliniques comprenant une partie inférieure et un couvercle débordant pouvant être monté sur cette partie inférieure et muni d'un bord rabattu et des orifices permettant l'échange entre les milieux air-vapeur pendant l'opération de stérilisation et comprenant des filtres ou des soupapes recouvrant ces orifices qui protègent le matériel stérilisé pendant le transport et pendant le stockage contre une nouvelle contamination, tandis que sur les orifices d'échange et respectivement les soupapes ou filtres, à une certaine distance de ceux-ci, est prévu un recouvrement de protection, un couvercle intermédiaire perforé étant disposé entre le couvercle du récipient et la partie inférieure, caractérisé par le fait que les orifices (16) et les filtres (18) ou les soupapes (20) sont disposés dans et respectivement sur le couvercle intermédiaire (14 ; 14a) qui peut être monté de manière étanche sur la partie inférieure du récipient (10), que le recouvrement de protection est formé par le couvercle (12) du récipient lui-même ne comportant aucune perforation et que dans la zone du bord (38) rabattu du couvercle, celui-ci est espacé du bord supérieur (30) de la partie inférieure (10), et respectivement du couvercle intermédiaire (14 ; 14a) de manière à former une fente annulaire (40) réalisant un trajet d'écoulement libre dans l'espace entre le couvercle (12) du récipient et le couvercle intermédiaire (14 ou 14a).

2. Récipient de stérilisation selon la revendication 1, caractérisé par le fait que le couvercle intermédiaire (14 ; 14a) est fixé de manière amovible sur le couvercle (12) du récipient à une certaine distance de celui-ci.

3. Récipient de stérilisation selon l'une des revendications 1 ou 2, caractérisé par le fait que le couvercle intermédiaire (14 ; 14a) présente une partie centrale munie de perforations (16) ou de soupapes (20), une partie de transition (22) s'étendant coniquemnt vers l'extérieur et vers le bas et une partie de bord (24) qui est montée de façon étanche par rapport au bord (30) de la partie inférieure du récipient (20).

4. Récipient de stérilisation selon la revendication 3, caractérisé par le fait qu'entre la partie de bord (24) du couvercle intermédiaire (14 ; 14a) et le bord (30) en déport vers l'intérieur de la partie inférieure (10) du récipient est disposée une bague d'étanchéité (28) qui est fixée sur le couvercle intermédiaire ou sur le bord du récipient.

5. Récipient de stérilisation selon l'une des revendications 1 à 4, caractérisé par le fait qu'un filtre (18) peut être monté sur la partie de bord (24) du couvercle intermédiaire (14).

6. Récipient de stérilisation selon la revendication 5, caractérisé par le fait que le couvercle (12) du récipient supporte, par l'intermédiaire de pièces d'écartement (36) tournées vers l'intérieur, un profilé en U (34) dans lequel est insérée une bague d'étanchéité (32) qui, à l'état monté, presse le bord du filtre (18) contre la partie de bord extérieure (24) du couvercle intermédiaire (14).

7. Récipient de stérilisation selon la revendication 5, caractérisé par le fait que le couvercle (12) du récipient supporte sur son côté interne une tôle élastique (44) périphérique comportant des orifices de passage (46), et qui s'appuie de manière élastique contre la partie de transition conique (22) du couvercle intermédiaire (14) et le bord du filtre (18) placée dessus.

8. Récipient de stérilisation selon la revendication 5, caractérisé par le fait que le couvercle (12) du récipient supporte à l'intérieur un cadre rigide périphérique (48) qui s'applique par un bord d'appui (50) contre la partie de bord (24) du couvercle intermédiaire (14) avec interposition du bord du filtre et d'un joint élastique plat (52).

9. Récipient de stérilisation selon la revendication 5, caractérisé par le fait que la partie centrale non perforée du couvercle intermédiaire (14a) supporte une soupape double (20).

# FIG.1

# FIG. 2

# FIG.3

# FIG.4

# FIG.5

FIG.7

FIG.8

FIG. 6

# FIG.9

# FIG.10

# FIG.11    FIG.12

FIG.13

FIG.14

FIG.15

FIG.16

FIG.17